(19) Europäisches Patentamt / European Patent Office / Office européen des brevets

(11) **EP 4 344 752 B1**

(12) **FASCICULE DE BREVET EUROPEEN**

(45) Date de publication et mention de la délivrance du brevet:
**25.06.2025 Bulletin 2025/26**

(21) Numéro de dépôt: **23187029.6**

(22) Date de dépôt: **21.07.2023**

(51) Classification Internationale des Brevets (IPC):
*A62B 18/08* (2006.01)   *F17C 11/00* (2006.01)
*A62B 19/00* (2006.01)   *A62B 18/00* (2006.01)
*C01B 13/02* (2006.01)   *F16K 1/00* (2006.01)
*A62B 7/00* (2006.01)

(52) Classification Coopérative des Brevets (CPC):
**A61M 16/22; A62B 7/00; A62B 9/006;
A62B 18/088; A62B 19/00; A62B 21/00;
C01B 13/027; F16K 15/025; F17C 11/00;**
A61M 16/0078; A61M 16/06; A61M 16/0891;
A61M 16/1005; A61M 16/201; A61M 2202/0208;
(Cont.)

(54) **ENSEMBLE DE DISTRIBUTION D'OXYGÈNE DE SECOURS INCLUANT UNE CARTOUCHE DE STOCKAGE D'OXYGÈNE**

NOTSAUERSTOFFABGABEANORDNUNG MIT SAUERSTOFFSPEICHERPATRONE

EMERGENCY OXYGEN DELIVERY ASSEMBLY INCLUDING AN OXYGEN STORAGE CARTRIDGE

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priorité: **28.09.2022 FR 2209855**

(43) Date de publication de la demande:
**03.04.2024 Bulletin 2024/14**

(73) Titulaire: **L'AIR LIQUIDE, SOCIETE ANONYME
POUR L'ETUDE ET
L'EXPLOITATION DES PROCEDES GEORGES
CLAUDE
75007 Paris (FR)**

(72) Inventeur: **BOULANGER, Thierry
75007 Paris (FR)**

(74) Mandataire: **Air Liquide
L'Air Liquide S.A.
Direction de la Propriété Intellectuelle
75, Quai d'Orsay
75321 Paris Cedex 07 (FR)**

(56) Documents cités:
WO-A1-2022/108320   WO-A1-95/12432
WO-A2-2012/018766   US-A1- 2021 121 649
US-B2- 7 875 100

(52) Classification Coopérative des Brevets (CPC):
    (Cont.)A61M 2205/0227; A61M 2205/584;
    A61M 2205/75; A61M 2205/7563; A62B 18/00

**Description**

La présente invention concerne une cartouche de stockage d'oxygène portative permettant une fourniture d'oxygène ($O_2$) à un utilisateur, pendant un temps suffisant, dans une situation particulière engendrant une atmosphère néfaste, hypoxique et/ou chargée de composés délétères, telle que dans les industries chimiques ou bien minières, laquelle cartouche comprenant en outre une ligne de bipasse contenant un adsorbant et un indicateur de saturation, et ensemble de distribution d'oxygène de secours comprenant une telle cartouche raccordée fluidiquement à un réservoir de gaz et à une interface respiratoire, tel un masque respiratoire.

L'administration d'$O_2$ gazeux est utilisé pour corriger une situation hypoxémique, c'est-à-dire lorsque la saturation d'oxyhémoglobine dans le sang est inférieure à une valeur normale, par exemple inférieure à 90%, chez une personne humaine, quel que soit son âge, i.e. nouveaux nés, enfants, adolescents, adultes, personnes âgées ou autres, souffrant une pathologie respiratoire du type Bronchopneumopathie Chronique Obstructive (BPCO), Syndrome de Détresse Respiratoire Aigüe (SDRA) ou autre.

Dans ce cas, l'oxygène de qualité médicale est fourni à la personne « hypoxique » soit au sein d'un l'hôpital, soit hors hôpital, par exemple dans une unité de secours mobile (SAMU, ambulances...) ou au domicile du patient. Selon le cas, l'oxygène fourni au patient peut provenir soit d'une canalisation de gaz (e.g. réseau hospitalier), soit d'une bouteille de gaz sous pression contenant par exemple de 400 à 1000 L d'$O_2$ (vol. gazeux) comprimé jusqu'à 200 bar abs ou plus (mesuré à 1 atm).

Il existe toutefois des situations particulières dans lesquelles un apport d'$O_2$ est nécessaire, avant même qu'une personne ne montre des signes d'hypoxémie. C'est en particulier le cas lorsqu'une personne est exposée à une atmosphère gazeuse « irrespirable », par exemple en présence de composés chimiques volatils toxiques au sein d'une pièce ou d'un bâtiment industriel ou bien en présence d'une atmosphère potentiellement hypoxique, tel que rencontré dans des zones confinées, comme par exemple les mines, les égouts ou bien également des cuves ayant contenu de l'azote ($N_2$) par exemple.

Dans ces situations particulières, les documents FR2201224 et FR2201227 (non publiés) proposent d'utiliser une cartouche de stockage d'oxygène portative comprenant un corps de cartouche fermé délimitant un volume interne recevant l'oxygène gazeux. Le corps de cartouche est raccordé fluidiquement à un réservoir de gaz et à une interface respiratoire, en particulier un masque respiratoire, tel un masque nasal ou facial, de sorte que des échanges gazeux puissent s'opérer entre ces éléments. Des valves de contrôle de flux gazeux servent à contrôler les entrées et sorties de gaz du volume interne. Le volume interne contient un lit d'adsorbant, par exemple de la zéolite, servant à piéger les molécules de $CO_2$ se trouvant dans le gaz expiré par un utilisateur et qui pénètrent dans le volume interne de la cartouche après avoir été récupéré part le masque respiratoire. Une telle cartouche de stockage d'oxygène se montre efficace pour fournir un apport d'oxygène de secours à une personne confrontée à situation particulière engendrant une atmosphère néfaste hypoxique (i.e. <21% $O_2$) et/ou chargée de composés délétères, comme les fumées en cas d'incendie ou analogue, pour lui permettre d'éviter de respirer cette atmosphère néfaste en lui fournissant de l'oxygène, pendant une durée de plusieurs dizaines de minutes.

Toutefois, en pratique, il est apparu un problème lié à la saturation de l'adsorbant contenant dans la cartouche par du $CO_2$ expiré par l'utilisateur. En effet, au fur et à mesure de l'utilisation de la cartouche, l'adsorbant se charge progressivement en $CO_2$ expiré par l'utilisateur et il est actuellement impossible de savoir quand l'adsorbant est totalement saturé puisque rien ne permet de distinguer visuellement un adsorbant saturé d'un adsorbant non-saturé et ce, d'autant moins que ce type de cartouche est formé d'un corps métallique totalement opaque.

Or, ceci pose un réel problème de sécurité pour l'utilisateur car, lorsque l'adsorbant est saturé, le $CO_2$ exhalé ne peut plus être retenu par cet adsorbant et va alors s'accumuler progressivement et se concentrer dans le réservoir de gaz, pouvant alors engendrer une intoxication gazeuse de l'utilisateur, i.e. une hypoxie, si celui-ci respire ce $CO_2$ accumulé sur une période longue, c'est-à-dire plusieurs minutes ou dizaines de minutes.

Dans ce contexte, un problème est alors de pouvoir connaître simplement le degré ou taux de saturation d'une telle cartouche de stockage d'oxygène afin d'éviter le problème susmentionné, lié à la saturation du lit d'adsorbant conduisant à une non-rétention du $CO_2$ exhalé lorsque l'adsorbant est totalement saturé. US2021121649A1 divulgue les caractéristiques du préambule de la revendication 1.

Une solution de l'invention concerne alors une cartouche de stockage d'oxygène portative comprenant :

- un corps de cartouche délimitant un volume interne servant au stockage de l'oxygène et comprenant un premier orifice de gaz et un second orifice de gaz en communication fluidique avec le volume interne,
- au moins une valve de contrôle de flux gazeux agencée dans chacun desdits premier et second orifices de gaz pour contrôler la circulation de gaz entrant dans le volume interne et/ou sortant du volume interne du corps de cartouche,
- et au moins un lit d'adsorbant contenant au moins un premier adsorbant étant agencé dans le volume interne du corps de cartouche, ledit premier adsorbant présentant une capacité d'adsorption pour le $CO_2$ gazeux supérieure à une capacité d'adsorption pour l'oxygène gazeux.

De plus, selon l'invention, la cartouche de stockage d'oxygène portative comporte en outre au moins une ligne de bipasse venant se raccorder fluidiquement en des sites de raccordement situés en amont de chacun desdits premier et second orifices de gaz de manière à ce qu'une partie du gaz entrant dans le volume interne par l'un ou l'autre desdits premier et second orifices de gaz soit dérivée et circule au sein de ladite au moins une ligne de bipasse. Ladite au moins une partie de ladite au moins une ligne de bipasse est formée d'au moins un matériau transparent et contient au moins un second matériau adsorbant et au moins un matériau indicateur de saturation.

Ainsi, une partie du flux gazeux contenant du $CO_2$ peut être déviée et circuler dans la ligne de bipasse. Le $CO_2$ est alors progressivement retenu sur le second matériau adsorbant, par exemple de la chaux sodée, ce qui engendre une variation du pH. Cette variation progressive de pH peut alors être « détectée » par le matériau indicateur de saturation, tel du violet d'éthyle, qui va alors changer progressivement de couleur en fonction de la quantité de $CO_2$ piégé sur le second matériau adsorbant, donc de sa saturation par le $CO_2$ piégé. Cette variation de couleur reflétant le taux de saturation du second matériau adsorbant peut être visualisée facilement et en temps réel par l'utilisateur au travers du matériau transparent constituant la ligne de bipasse de sorte de lui indiquer lorsque le second matériau adsorbant est totalement saturé et donc la cartouche n'est plus opérationnelle et doit être remplacée.

Selon le mode de réalisation considéré, l'invention peut comprendre l'une ou plusieurs des caractéristiques suivantes :

- le matériau transparent est un polymère, de préférence un thermoplastique.
- le polymère est un polycarbonate.
- la ligne de bipasse comprend un tube allongé.
- le tube allongé est cylindrique.
- le tube allongé a un diamètre compris entre 0.5 et 10 mm, de préférence de l'ordre de 5 mm.
- ledit au moins un premier adsorbant comprend des particules d'au moins une zéolite.
- ledit au moins un second matériau adsorbant contenu dans la ligne de bipasse comprend de la chaux sodée.
- la chaux sodée comprend majoritairement de l'hydroxyde de calcium ($CaOH)_2$, typiquement d'au moins 50 à 90% environ en poids d'hydroxyde de calcium.
- la chaux sodée peut comprendre en outre de l'hydroxyde de sodium (NaOH), typiquement moins de 10% en poids d'hydroxyde de sodium.
- le matériau indicateur de saturation est configuré pour changer progressivement de coloration en fonction de son degré de saturation par du $CO_2$.
- le matériau indicateur de saturation est configuré pour changer de coloration en fonction de son degré de saturation par du $CO_2$ en passant progressivement d'une coloration blanchâtre à une coloration violette.
- le matériau indicateur de saturation comprend de l'éthyle violet, aussi appelé violet d'éthyle.
- le matériau indicateur de saturation est sous forme particulaire, de préférence sous forme de granulés ou analogue.
- le matériau indicateur de saturation est sensiblement au pH, c'est-à-dire qu'il réagit en changeant de couleur (i.e. teinte ou colorisation) en fonction du pH auquel il est soumis.
- le second adsorbant, en particulier de la chaux sodée, est sous forme particulaire, de préférence sous forme de granulés ou analogue.
- lesdits au moins un second matériau adsorbant et ledit au moins un matériau indicateur de saturation sont mélangés l'un à l'autre dans la ligne de bipasse.
- la ligne de bipasse comprend un mélange particulaire contenant de l'hydroxyde de calcium ($CaOH)_2$ et du violet d'éthyle, et de préférence du chlorure de lithium (LiCl).
- la ligne de bipasse comprend des granules comprenant majoritairement de l'hydroxyde de calcium ($CaOH)_2$, i.e. > 95% en poids environ, du chlorure de lithium (LiCl), i.e. <3% en poids environ, et de l'éthyle violet, i.e. environ 1% en poids.
- la ligne de bipasse comprend au moins 95% en poids environ d'hydroxyde de calcium ($CaOH)_2$, moins de 3% en poids environ de chlorure de lithium (LiCl), et jusqu'à environ 1 % en poids d'éthyle violet.
- les particules de chaux sodée piègent, c'est-à-dire retiennent, captent ou analogue, le $CO_2$ présent dans le flux gazeux, i.e. le gaz expiré par l'utilisateur.
- le matériau indicateur de saturation réagit en changeant progressivement de couleur au fur et à mesure du piégeage (i.e. rétention) du $CO_2$ par les particules de chaux sodée engendrant une variation du pH.
- le matériau indicateur de saturation réagit en changeant progressivement de couleur en passant d'une couleur blanchâtre à une couleur violette.
- le matériau transparent est un polymère, de préférence un thermoplastique.
- le matériau transparent est un polycarbonate.
- le matériau transparent est choisi pour permettre à l'utilisateur de distinguer le changement de couleur du matériau indicateur de saturation contenu dans la ligne de bipasse.
- la ligne de bipasse vient de raccorder à un premier et à un second conduits.

- lesdits premier et second conduits sont raccordés au corps de cartouche et en communication fluidique avec le volume interne du corps de cartouche, via les premier et second orifices de gaz, respectivement.
- lesdits premier et second conduits sont raccordés au corps de cartouche à l'extérieur dudit corps de cartouche, c'est-à-dire en amont des premier et second orifices de gaz.
- la première valve est agencée au niveau du premier orifice de gaz de manière à contrôler tout passage de gaz entrant ou sortant du volume interne via le premier orifice de gaz.
- la second valve est agencée au niveau du second orifice de gaz de manière à contrôler tout passage de gaz entrant ou sortant du volume interne via le second orifice de gaz.
- la ligne de bipasse est raccordée aux premier et second conduits via un premier et un second conduit de dérivation.
- la ligne de bipasse comprend des tamis, telles des grilles ou analogues, prenant en sandwich le second adsorbant et le matériau indicateur de saturation.
- la ligne de bipasse comprend un marquage gradué, c'est-à-dire des graduations ou analogues.
- le corps de cartouche est de forme tubulaire.
- le corps de cartouche est fermé à deux extrémités opposées par un premier et un second couvercle.
- le corps tubulaire et les premier et second couvercles délimitent le volume interne servant au stockage de l'oxygène.
- les valves de contrôle de flux gazeux sont agencées sur lesdits premier et second couvercles, en particulier une première valve de contrôle de flux gazeux est agencée sur le premier couvercle et une seconde valve de contrôle de flux gazeux est agencée sur le second couvercle.
- le volume interne comprend une chambre centrale contenant ledit au moins un lit d'adsorbant dudit au moins un premier adsorbant.
- le volume interne comprend en outre une première chambre de recueil de gaz et une seconde chambre de recueil de gaz, la chambre centrale étant située entre les première et seconde chambres de recueil de gaz, c'est-à-dire prise en sandwich entre lesdites première et seconde chambres de recueil de gaz.
- le corps de cartouche est hermétique.
- le corps de cartouche communique fluidiquement avec l'extérieur via les premier et second orifices de gaz et au travers des valves de contrôle de flux gazeux.
- la chambre centrale est séparée de la première et de la seconde chambre de recueil de gaz par une première et une seconde structure de tamis, respectivement.
- les première et seconde structure de tamis comprennent des grilles ou analogues.
- les grilles ont une forme de disque.
- le corps tubulaire a une section interne et les structures de tamis sont agencées radialement dans le volume interne, i.e. perpendiculairement à l'axe principal (XX), de manière à s'étendre sur toute la section interne du corps tubulaire.
- le corps tubulaire est cylindrique.
- le volume intérieur de la cartouche est inférieur à 1 L, typiquement de l'ordre de 300 ml à 900 ml (équiv. en eau).
- les structures de tamis sont configurées pour maintenir le premier lit d'adsorbant contenant ledit premier adsorbant.
- ledit au moins un premier adsorbant comprend des particules d'au moins une zéolite.
- le premier lit d'adsorbant contient des particules de zéolite 13X ou 5A présentant une capacité d'adsorption pour l'oxygène d'au moins 3 ml/g à 1 bar et 20°C.
- ledit au moins un second matériau adsorbant contenu dans la ligne de bipasse comprend de la chaud sodée.
- le premier adsorbant et le second adsorbant sont configurés ou choisis pour qu'un front de saturation en $CO_2$ s'y déplace progressivement au fur et à mesure que du $CO_2$ y est retenu.
- le dimensionnement de la ligne de bipasse et la masse de second adsorbant sont configurés et/ou choisis pour que la progression du front de saturation en $CO_2$ soit identique dans le premier adsorbant, de préférence une zéolite, et dans le mélange de second adsorbant, telle de la chaux sodée, et de matériau indicateur de saturation.
- chaque valve de contrôle comprend un corps de valve, un moyen d'étanchéité annulaire, un élément mobile et un moyen élastique coopérant les uns avec les autres pour contrôler le passage de gaz au travers de chaque valve de contrôle.
- chaque valve de contrôle comprend en outre une pièce-couvercle venant se fixer au corps de valve.
- la pièce-couvercle vient se fixer par vissage au corps de valve de chaque valve de contrôle.
- la pièce-couvercle porte un filetage périphérique externe et le corps de valve de chaque valve de contrôle porte un taraudage interne complémentaire du filetage périphérique externe de la pièce-couvercle.
- chaque valve de contrôle est mobile entre une position de repos et une position actionnée ou active.
- en position de repos, aucun gaz ne peut entrer ou sortir de la cartouche par l'une et/ou l'autre des valves de contrôle.
- en position actionnée ou active, un flux de gaz peut s'établir au travers de l'une et/ou l'autre des valves de contrôle de manière entrer ou sortir de la cartouche via l'une et/ou l'autre des valves de contrôle.
- le moyen élastique est agencé dans la pièce-couvercle.
- le moyen élastique est pris en sandwich entre la pièce-couvercle et l'élément mobile.
- le moyen élastique est maintenu par des bossages agencés sur le fond de la pièce-couvercle et sur la paroi extérieure du fond de l'élément mobile.
- l'élément mobile comprend une collerette périphérique.
- le moyen élastique repousse l'élément mobile contre le moyen d'étanchéité annulaire.
- le moyen élastique repousse l'élément mobile contre le moyen d'étanchéité annulaire de sorte que la collerette périphérique de l'élément mobile vienne au contact de le moyen d'étanchéité lorsqu'une valve est en position de repos.
- le moyen élastique est un ressort à spires cylindrique.
- le moyen d'étanchéité annulaire est un joint torique plat.
- le corps de valve comprend un fond annulaire présentant un évidement central, le moyen d'étanchéité annulaire étant agencé sur ledit fond annulaire, coaxialement audit évidement central.
- l'élément mobile comprend une collerette coopérant avec le moyen d'étanchéité annulaire pour assurer une étanchéité fluidique et empêcher toute circulation de gaz au travers de l'évidement central du corps de valve de l'une et/ou l'autre des valves de contrôle, lorsque l'une et/ou l'autre des valves de contrôle est/sont en position de repos.
- en position active, la collerette de l'élément mobile n'est pas ou plus au contact du moyen d'étanchéité annulaire de sorte que étanchéité fluidique soit rompue et qu'une circulation de gaz au travers de l'évidement central du corps de valve de l'une et/ou l'autre des valves de contrôle puisse s'établir.
- le corps de valve, le moyen d'étanchéité annulaire, l'élément mobile, le moyen élastique et la pièce-couvercle de chaque valve de contrôle sont coaxiaux (axe XX).
- le corps de valve de chaque valve de contrôle a une forme tubulaire, typiquement cylindrique.
- le corps de valve comprend une collerette annulaire se projetant radialement vers l'extérieur.
- la collerette annulaire est agencée au niveau d'une première extrémité du corps de valve.
- le corps de valve de chaque valve de contrôle comprend intérieurement un fond annulaire, de préférence plat.
- le fond annulaire du corps de valve présente en son centre, un évidement circulaire.
- le fond annulaire du corps de valve forme un épaulement interne s'étirant le long de la paroi interne, i.e. du périmètre interne, du corps de valve.
- l'élément mobile est mobile en translation axiale (axe XX) dans le corps de valve de chaque valve.
- l'élément mobile a une forme de coupelle.
- l'élément mobile comprend une ou plusieurs ouvertures aménagées dans sa paroi périphérique.
- la pièce-couvercle a une forme de coupelle.
- la pièce-couvercle comprend une ou plusieurs ouvertures aménagées dans sa paroi périphérique.
- la paroi périphérique de la pièce-couvercle a une forme cylindrique.
- l'élément mobile de chaque valve de contrôle comprend une collerette périphérique externe comprenant un ou plusieurs logements ou évidements, telles des encoches.
- la pièce-couvercle comprend un ou des guides axiaux, tels des rails de guidage ou analogues, aménagés axialement le long de sa paroi interne.
- le ou les évidements portés par la collerette périphérique externe de l'élément mobile sont conformés pour loger les guides axiaux de la pièce-couvercle de sorte que ces guides axiaux puissent guider un déplacement translatif de la pièce mobile dans la pièce-couvercle.

L'invention concerne en outre un ensemble de distribution d'oxygène de secours comprenant une cartouche de stockage d'oxygène portative pour stocker de l'oxygène selon l'invention, telle que décrite ci-avant, raccordée fluidiquement via lesdits premier et second orifices de gaz de la cartouche à un réservoir de gaz et à une interface respiratoire.

De préférence, le réservoir de gaz est à enveloppe flexible et/ou l'interface respiratoire est un masque respiratoire, en particulier un masque nasal ou facial.

L'invention va maintenant être mieux comprise grâce à la description détaillée suivante, faite à titre illustratif mais non limitatif, en référence aux figures annexées parmi lesquelles :

Fig. 1 est une vue schématique en coupe d'un mode de réalisation d'une cartouche de stockage d'oxygène telle que décrite par FR2201224 ou FR2201227,
Fig. 2 est une vue éclatée d'une des valves intégrées à la cartouche de stockage d'oxygène de Fig. 1,
Fig. 3 est une vue éclatée d'une partie de la valve de Fig. 2,
Fig. 4 est une vue latérale et en coupe de la valve de Fig. 2, dans sa position de « repos »,
Fig. 5 est une vue en coupe de la valve de Fig. 2, dans sa position « actionnée »,
Fig. 6 est une vue latérale de la valve de Fig. 5,
Fig. 7 illustre la cartouche de stockage d'oxygène de Fig. 1, avec ses valves en position « actionnée »,
Fig. 8 schématise la cartouche de stockage d'oxygène de Fig. 1, raccordée à un réservoir (partiellement montré) et à un masque (non montré), via des conduits de gaz,

Fig. 9 schématise un ensemble de distribution d'oxygène de secours comprenant la cartouche de stockage d'oxygène de Fig. 1, un réservoir pour l'oxygène et un masque respiratoire,

Fig. 10 schématise un mode de réalisation d'une cartouche de stockage d'oxygène améliorée selon la présente invention,

Fig. 11 est une vue en coupe simplifiée de la cartouche selon Fig. 10, et

Fig. 12 schématise un mode de réalisation d'un ensemble de distribution d'oxygène de secours selon l'invention, comprenant la cartouche de stockage d'oxygène de Fig. 10 et Fig. 11.

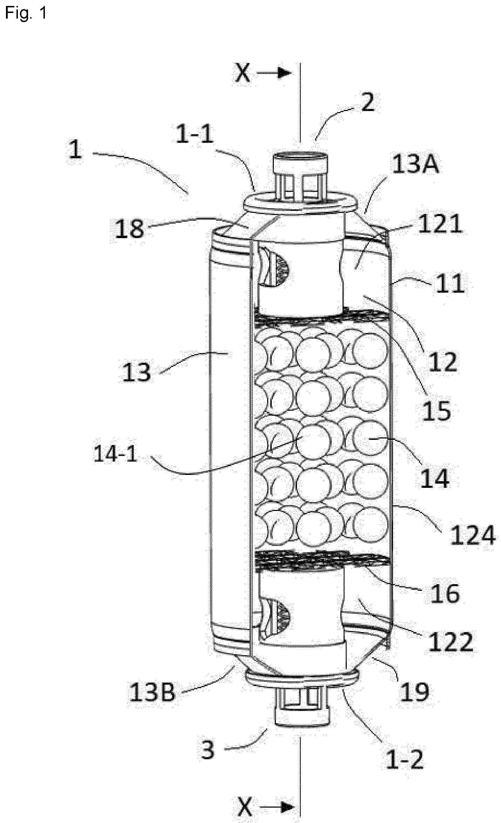

Fig. 1 est une vue schématique en coupe d'un mode de réalisation d'une cartouche 1 de fourniture d'oxygène de secours, telle que décrite par FR2201224 ou FR2201227.

La cartouche 1 comprend un corps de cartouche ou corps principal 13 fermé à ses deux extrémités opposées 13A, 13B par un premier couvercle 18 et un second couvercle 19, telles des parois ou analogues fermant des deux extrémités 13A, 13B du corps principal 13, de manière à délimiter un volume interne 12 servant au stockage du gaz, à savoir ici de l'oxygène ($O_2$) sous forme gazeuse.

Sont prévus en outre un premier orifice de gaz 1-1 et un second orifice de gaz 1-2 qui communiquent fluidiquement avec le volume interne 12. Ils sont agencés de part et d'autre du corps principal 13, c'est-à-dire que le premier orifice de gaz 1-1 est aménagé dans le premier couvercle 18, alors que le second orifice de gaz 1-2 est aménagé dans le second couvercle 19. Une première valve 2 et une seconde valve 3 de contrôle de flux sont agencées dans ces premier orifice de gaz 1-1 et second orifice de gaz 1-2, respectivement, pour contrôler les entrées et les sorties de gaz, c'est-à-dire les flux de gaz traversant le corps de cartouche 13, comme expliqué ci-après.

Le corps principal 13 est formé ici d'une enveloppe tubulaire 11 externe, préférentiellement de forme cylindrique. Le corps principal 13 est de préférence en métal, par exemple en un alliage d'aluminium. L'enveloppe 11 tubulaire est allongée et s'étend selon un axe principal XX entre les deux extrémités 13A, 13B.

Le premier couvercle 18 et le second couvercle 19 portant les orifices de gaz 1-1, 1-2, prennent le corps cylindrique 13, typiquement l'enveloppe 11 tubulaire, en « sandwich » et y sont fixés de manière étanche, par exemple soudés ou sertis.

Les premier et le second couvercle 18, 19 sont préférentiellement aussi en métal, par exemple en alliage d'aluminium.

La paroi de l'enveloppe périphérique 11 formant le corps principal 13 est fine, c'est-à-dire qu'elle a une épaisseur de l'ordre de quelques dixièmes de millimètres.

Par ailleurs, le volume intérieur de la cartouche 1 est de l'ordre de 300 ml à 900 ml (équiv. en eau).

Un (ou plusieurs) lit(s) 14 d'un (ou plusieurs) premier adsorbant particulaire 14-1, telles des billes d'adsorbant par exemple, est agencé dans le volume intérieur 12. En particulier, le lit de premier adsorbant 14-1 est pris en sandwich et maintenu par/entre deux structures de tamis 15, 16, plus simplement appelées 'tamis', c'est-à-dire que le premier tamis 15 et le second tamis 16 sont disposés de part et d'autre du lit d'adsorbant 14 de manière à le maintenir en position dans le volume interne 12 de la cartouche 1.

Autrement dit, les structures de tamis 15, 16 qui sont agencées radialement dans le volume interne 12, c'est-à-dire perpendiculairement à l'axe principal XX, et s'étendent sur toute la section interne du corps principal 13 de forme cylindrique.

Préférentiellement, les structures de tamis 15, 16 permettent de séparer le volume interne 12 en plusieurs chambres ou compartiments internes, à savoir une chambre centrale 124 contenant le premier lit 14 de premier adsorbant 14-1 située au centre de la cartouche 1, et une première et une seconde chambre de recueil de gaz 121, 122 situées aux extrémités opposées 13A, 13B, c'est-à-dire entre la chambre centrale 124 et les premier et second couvercle 18, 19.

Les tamis 15, 16 sont par exemple formés d'un maillage de fibres métalliques présentant une multitude de pores dont les dimensions sont inférieures aux dimensions du premier adsorbant 14-1. Selon d'autres modes de réalisation, les tamis 15, 16 peuvent se présenter sous forme de mousses comprenant ou formées d'alvéoles interconnectées, ou encore sous forme de grilles, par exemple de grilles métalliques, ou analogues.

Dans tous les cas, les tamis 15, 16 sont dimensionnés pour retenir les particules d'adsorbant du premier lit 14 de premier adsorbant 14-1. Autrement dit, quel que soit le mode de réalisation choisi, le lit 14 de premier adsorbant 14 est « piégé » et maintenu entre les deux tamis 15, 16, alors que les molécules de gaz sont libres de circuler à travers eux.

En outre, le premier adsorbant 14-1 est choisi pour permettre d'adsorber les molécules de gaz, en particulier celles d'oxygène, du fait de minuscules pores à sa surface, c'est-à-dire des pores de l'ordre du ou de quelques nanomètre (nm). Ainsi, il peut être choisi parmi les charbons actifs, les structures organométalliques ou MOF pour « *Metal Organic Framework* » ou les zéolites. Toutefois, on utilise, de préférence, un adsorbant zéolitique, c'est-à-dire une ou plusieurs zéolites, se présentent sous forme de particules, telles des granules, des billes ou analogues, d'une (ou plusieurs) zéolite ayant une capacité d'adsorption élevée, c'est-à-dire d'au moins 3 ml/g à 1 bar et 20°C, pour l'oxygène, par exemple une zéolite 13X ou 5A, par exemple la zéolite référencée Z10-ZZ commercialisée par la société Zeochem®.

Comme visible sur Fig. 1, le lit d'adsorbant 14 ne remplit pas l'intégralité du volume interne 12 de la cartouche 1 mais est agencé uniquement dans la chambre centrale 124 de sorte qu'il existe des espaces vides aux deux extrémités 13A, 13B du volume interne 12 formant les première 121 et seconde 122 chambres de recueil de gaz.

La première chambre 121 de recueil de gaz est située entre le premier couvercle 18 portant le premier orifice de gaz 1-1 et le premier tamis 15, et la seconde chambre 122 de recueil de gaz est située entre le second couvercle 19 portant le second orifice de gaz 1-2 et le second tamis 16. Autrement dit, les deux tamis 15, 16 séparent la chambre centrale 124 contenant le premier lit d'adsorbant 14, des première et seconde chambres de recueil de gaz 121, 122 qui sont situées de part et d'autre de ladite chambre centrale 124, c'est-à-dire qui la prennent en sandwich en étant séparées de celle-ci par les tamis 15, 16.

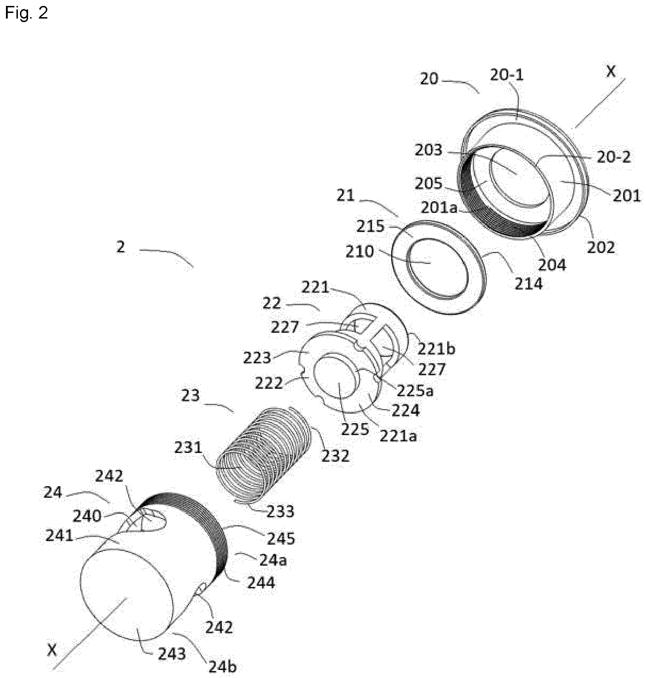

De plus, comme visible sur Fig. 1, la cartouche 1 d'oxygène gazeux selon l'invention présente, comme déjà dit, aussi une première valve 2 et une seconde valve 3 de contrôle de flux agencées à ses deux extrémités opposés 13A, 13B, typiquement portées par les couvercles 18, 19 et agencées au niveau des premier et second orifices de gaz 1-1, 1-2. Les première et seconde valves de contrôle de flux 2, 3 servent à contrôler la circulation de gaz, c'est-à-dire les flux gazeux entrant et sortant du volume interne 12 de la cartouche 1, via les premier et second orifices de gaz 1-1, 1-2 et au travers desdites première et seconde valves de contrôle de flux 2, 3, comme expliqué ci-après et illustré sur Fig. 2 à Fig. 8. Elles sont fixées de manière étanche, par exemple soudées ou serties, aux couvercles 18, 19, typiquement elles traversent les couvercles 18, 19. Les première et seconde valves 2, 3 de contrôle de flux sont préférentiellement mais pas obligatoirement identiques.

Un mode de réalisation de l'architecture de ces valves 2, 3 est détaillé ci-après en lien avec Fig. 2 à Fig. 5.

Fig. 2 est vue éclatée de la première valve 2 équipant la cartouche 1 de stockage d'oxygène de l'invention schématisée en Fig. 1, sachant que la seconde valve 3 lui est strictement identique et fonctionne de la même façon (la valve 3 n'est donc pas détaillée ci-après).

Plus précisément, la première valve 2, tout comme la deuxième valve 3, est formée d'un assemblage de plusieurs éléments 20-24 coopérant les uns avec les autres, comprenant un corps de valve 20, un moyen d'étanchéité annulaire 21, un élément mobile 22, un moyen élastique 23, tel un ressort cylindrique à spires, et une pièce-couvercle 24, qui sont détaillés ci-après.

Ces éléments 20-24 sont agencés coaxialement les uns par rapport aux autres par rapport à l'axe XX des valves 2, 3 et de la cartouche 1. De préférence, les éléments 20-24 sont métalliques, par exemple en un alliage d'aluminium ; bien entendu, d'autres matériaux adaptés peuvent aussi convenir.

**Corps de valve 20**

Le corps de valve 20 est de forme générale cylindrique. Il a une longueur de l'ordre de 8 à 16 mm mesurée entre ses deux extrémités 20-1, 20-2, et un diamètre interne (Di) typiquement compris entre 18 et 30 mm.

La paroi périphérique 201 du corps de valve 20 porte, au niveau de sa première extrémité 20-1, une collerette annulaire 202 se projetant radialement vers l'extérieur.

Autrement dit, la collerette annulaire 202 est solidaire de la paroi périphérique 201 externe cylindrique du corps de valve 20. Elles peuvent être formées d'une seule pièce ou de plusieurs pièces assemblées les uns aux autres.

La collerette 202 forme un épaulement externe, s'étirant sur toute la périphérie externe ou périmètre du corps de valve 20, permettant d'assurer une étanchéité fluidique entre le corps de valve 20 et le couvercle 18 intégrant la première valve 2 au sein du premier orifice 1-1, par exemple en y étant fixé par sertissage, soudage ou toute autre technique adaptée.

Par ailleurs, le corps de valve 20 présente aussi intérieurement un fond annulaire 205, de préférence plat. Le fond annulaire 205 présente en son centre, un évidement circulaire 203, et forme un épaulement interne, s'étirant le long de la paroi interne ou périmètre interne du corps de valve 20.

De plus, le corps de valve 20 porte un taraudage 204 aménagé sur une partie 201a de sa paroi interne 201, à partir de sa seconde extrémité 20-2. Ce taraudage 204 permet le raccordement par vissage de l'élément support 24, comme expliqué ci-après.

**Moyen d'étanchéité annulaire 21**

La première valve 2 comprend par ailleurs un moyen d'étanchéité annulaire 21, à savoir ici un joint annulaire plat, e.g. un joint torique.

De préférence, il a une épaisseur d'environ 1 mm et un diamètre externe (De) légèrement inférieur au diamètre interne (Di) du corps de valve 20, i.e. De<Di, de manière à pouvoir être inséré dans le corps de valve 20 et venir se positionner sur l'épaulement interne formé par le fond annulaire 205 du corps de valve 20.

Le joint annulaire plat 21 comprend une face avant 214 orientée vers le fond annulaire 205 du corps de valve 20 et une face arrière 215 opposée à la face avant 214, c'est-à-dire orientée en direction de la collerette 222 de l'élément mobile 22, comme détaillé ci-après. De plus, il présente par ailleurs un passage central 210 dont le diamètre est supérieur à celui de l'évidement circulaire 203 du corps de valve 20.

Il peut être constitué de différents matériaux, en particulier un élastomère, par exemple le caoutchouc nitrile, aussi appelé caoutchouc acrylonitrile butadiène ou caoutchouc NBR pour « *Nitrile Butadiene Rubber »* en anglais.

**Elément mobile 22**

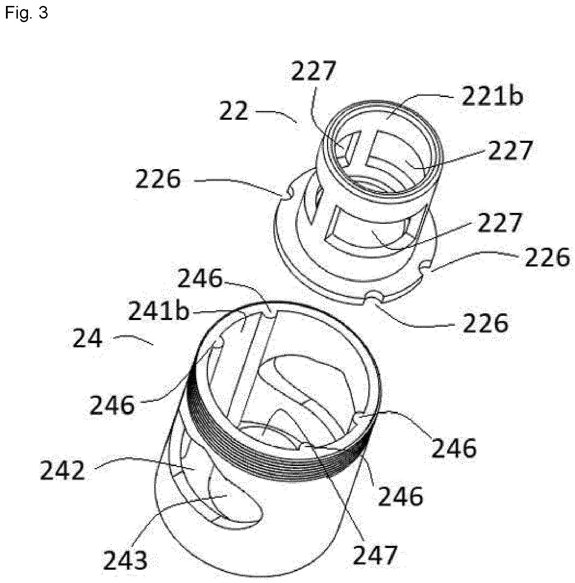

Comme visible sur Fig. 2 et Fig. 3, l'élément mobile 22 est une structure ayant une forme générale de coupelle. Plus précisément, l'élément mobile 22 comprend une portion avant 221 de forme cylindrique qui est ouverte à son extrémité distale 221b et obturée, c'est-à-dire fermée, à son extrémité proximale 221a par une paroi de fond 223, par exemple en forme de disque.

Préférentiellement, la paroi de fond 223 se projette radialement vers l'extérieur, c'est-à-dire en éloignement par rapport à la surface périphérique externe de la portion avant 221 de forme cylindrique de manière à former une collerette 222, i.e. une structure en anneau, s'étendant tout autour de l'extrémité proximale 221a cylindrique de la portion avant 221.

La portion cylindrique 221 a un diamètre externe inférieur au diamètre interne de l'évidement circulaire 203 du corps de valve 20, alors que la collerette 222 a un diamètre externe supérieur à celui de l'évidement 203 du corps de valve 20 de sorte que l'élément mobile 22 soit mobile axialement (axe XX) au travers de l'évidement circulaire 203 du corps de valve 20 et de l'évidement central 210 du joint annulaire plat 21 mais ne puisse pas totalement en sortir, étant donné qu'il y est retenu par la collerette 222 lorsque celle-ci vient buter sur le joint annulaire plat 21, qui est lui-même agencé sur le fond annulaire 205 du corps de valve 20, c'est-à-dire pris en sandwich entre le fond annulaire 205 du corps de valve 20 et la collerette 222 de l'élément mobile 22.

La paroi périphérique de la portion avant 221 cylindrique est ajourée, c'est-à-dire qu'elle présente plusieurs ouvertures latérales 227.

Par ailleurs, la face extérieure 224 de la paroi de fond 223 comprend un bossage central 225, c'est-à-dire un élément en relief, une excroissance de surface ou analogue, typiquement de section circulaire. Ici, le bossage central 225 est de forme générale cylindrique et a une hauteur de l'ordre de 1 mm et un diamètre qui est inférieur au diamètre de la collerette 222, comme visible en Fig. 2, par exemple un diamètre de l'ordre de 4 à 8 mm. Lorsque le moyen élastique 23 est un ressort à spires cylindrique délimitant un passage central cylindrique 231, le diamètre du bossage central 225 est légèrement inférieur à celui du passage central 231 du ressort de manière à ce que le ressort puisse venir s'insérer sur le bossage central 225 à la manière d'un manchon. Le bossage central 225 coopère avec le moyen élastique 23, comme expliqué ci-après.

**Moyen élastique 23**

Le moyen élastique 23 est préférentiellement un ressort à spires cylindrique venant se positionner autour du bossage central 225, tel un manchon, et appuyer sur la face extérieure 224 de la paroi de fond 223 dans une zone sensiblement annulaire située immédiatement autour du bossage central 225 de l'élément mobile 22. Le moyen élastique 23, i.e. le ressort, comprend une extrémité avant 232 venant au contact de l'élément mobile 22 et une extrémité arrière 233 venant au contact de pièce-couvercle 24.

Le diamètre interne du passage central 231 du ressort à spires est dès lors légèrement supérieur au diamètre du bossage central 225, comme déjà expliqué.

Le moyen élastique 23 repousse élastiquement l'élément mobile 22 en direction du moyen d'étanchéité 21.

**Pièce-couvercle 24**

La première valve 2 comprend par ailleurs une pièce-couvercle 24 tubulaire délimitant un logement interne 240 pour loger (au moins) le moyen élastique 2 qui est comprimé et va alors repousser axialement (XX) l'élément mobile 22.

Comme visible sur Fig. 2 et Fig. 3, la pièce-couvercle 24 est formée d'un corps principal 241 de forme cylindrique dont la paroi est percée, c'est-à-dire traversée, par une ou des ouvertures ou fenêtres latérales 242. Le corps principal 241 comprend, à son extrémité avant 24a, une ouverture 245 qui communique avec le logement interne 240. A son extrémité arrière 24b, le corps principal est fermé par une paroi formant un fond 243, de préférence un fond plat. Autrement dit, la pièce-couvercle 24 a une forme générale de coupelle ou similaire avec des ouvertures latérales 242.

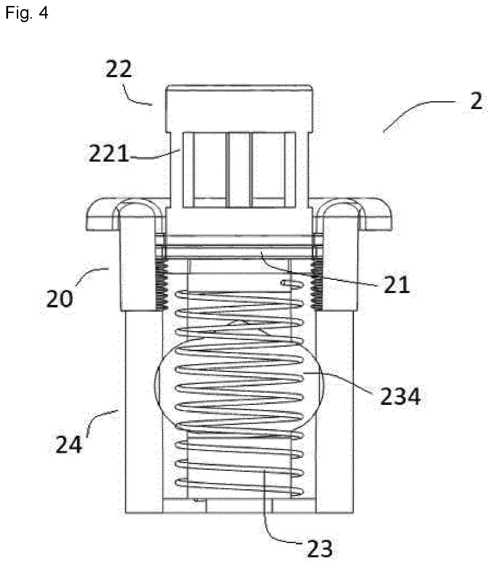

Le corps principal 241 de la pièce-couvercle 24 présente par ailleurs, sur sa surface extérieure, du côté de son extrémité avant 24a, un filetage 244 périphérique qui est complémentaire du taraudage 204 du corps de valve 20 pour que la pièce couvercle 24 puisse venir se fixer par vissage audit corps de valve 20 en prenant en 'sandwich' le moyen élastique 23, l'élément mobile 22 et le moyen d'étanchéité 21, comme visible sur Fig. 4.

Comme visible sur Fig. 2 et Fig. 4, le moyen d'étanchéité, c'est-à-dire le joint plat 21, est inséré dans le corps de valve 20. Sa face avant 214 vient en contact avec le fond annulaire 205 qui forme un épaulement interne du corps de valve 20.

L'évidement circulaire 203 situé au centre du fond annulaire 205 du corps de valve 20 est en communication fluidique avec le passage central 210 du joint plat 21.

Comme illustré en Fig. 4, lorsque la pièce-couvercle 24 est visée sur le corps de valve 20, elle agit sur le moyen élastique 23, à savoir le ressort à spires cylindrique, qui est alors comprimé et repousse alors, à son tour, l'élément mobile 22 en direction du corps de valve 20. L'élément mobile 22 se déplace alors axialement jusqu'à ce que sa portion avant 221 de forme cylindrique passe au travers du passage central 210 du joint plat 21 et de l'évidement circulaire 203 situé au centre du fond annulaire 205 du corps de valve 20 jusqu'à faire saillie hors du corps de valve 20. Le mouvement translatif de l'élément mobile 22 est stoppé lorsque la collerette 222 vient en butée contre la face arrière 215 du joint plat 21.

Le joint plat 21 se retrouve alors pris en « sandwich » entre le fond plat 205 du corps de valve 1 et la collerette 222 de la pièce mobile 22 qui est repoussée par le ressort cylindrique 23.

Plus précisément, l'extrémité avant 232 du ressort cylindrique 23 vient en contact avec la face extérieure 224 de la paroi de fond 223 de l'élément mobile 22 pour le repousser élastiquement axialement vers le joint plat 21, c'est-à-dire en direction du corps de valve 1.

L'extrémité arrière 233 du ressort cylindrique 23 est quant à elle en contact avec la surface interne de la paroi de fond 243 de la pièce-couvercle 24, de préférence elle vient de placer autour d'un bossage interne 247 faisant saillie au centre de la surface interne de la paroi de fond 243 de manière à opérer un maintien mécanique de l'extrémité arrière 233 du ressort cylindrique 23, comme expliqué ci-avant pour le bossage 225. Le ressort 23 est donc maintenu par et entre les bossages interne 247 de la pièce-couvercle 24 et externe 225 de la pièce mobile 22.

Par ailleurs, comme illustré en Fig. 3, préférentiellement, des logements ou évidements, telles des encoches 226, sont aménagés dans la collerette 222 de la pièce mobile 22 et des guides axiaux 246, i.e. des rails de guidage ou analogue, sont aménagés axialement le long de la paroi interne de la pièce-couvercle 24. Les encoches 226 sont conformées pour loger lesdits guides axiaux 246 de sorte que ces guides axiaux 246 puissent guider les déplacements translatifs de la pièce mobile 22 dans la pièce-couvercle 24.

Autrement dit, grâce à ces encoches 226 et guides axiaux 246, la pièce mobile 22 peut mieux coulisser dans le volume interne de la pièce-couvercle 24, en particulier en fonction du taux de compression du ressort 23.

Dans le mode de réalisation de Fig. 3, on voit 4 encoches 226 aménagées dans la collerette 222 de la pièce mobile 22 coopérant avec 4 guides axiaux 246 correspondants aménagés axialement le long de la paroi interne de la pièce-couvercle 24.

Fig. 4 est une vue latérale et en coupe de la valve 2 de Fig. 2, dans sa position de « repos », c'est-à-dire libre de toute contrainte externe.

Dans ce cas, le ressort 23 est comprimé (zone 234) entre la collerette 222 de la pièce mobile 22 et le fond de la pièce-couvercle 24, et la pièce mobile 22 repousse alors le joint plat 21 contre le fond plat 205 du corps de valve 20 de manière à obtenir une étanchéité fluidique entre eux.

Dans cette position de « repos », aucun échange de gaz entre l'intérieur et l'extérieur de la cartouche 1 ne se produit au travers des valves 2, 3 étant donné qu'elles sont « fermées » étant donné que l'orifice 203 de chaque corps de valve 20 est alors obturé par la paroi de fond 223 de la pièce mobile 22.

La cartouche 1 est alors étanche et son volume interne 12 peut alors stocker de l'oxygène sous pression. Ainsi, avant utilisation, la cartouche 1 peut être remplie d'$O_2$ à une pression de quelques bars, par exemple de l'ordre d'au maximum 10 bar absolus, ce qui permet de répondre aux contraintes de logistiques, notamment d'acheminement et d'entreposage. L'introduction de l'$O_2$ dans l'enveloppe 11 peut se faire via une valve de remplissage (non montrée) agencée par exemple sur le couvercle 18 ou préférentiellement, via l'une des valves 2,3, c'est-à-dire au travers de l'un ou l'autre des orifices 1-1, 1-2 qui reçoivent les première et seconde valves de contrôle de flux 2, 3.

La présence du premier adsorbant 14-1 dans le volume interne 12 de la cartouche 1 permet de stocker une quantité d'oxygène très supérieure à celle que pourrait contenir ce récipient de gaz 1 en l'absence d'adsorbant. Préférentiellement, on utilise en tant que premier adsorbant 14-1, une zéolite ayant une forte capacité d'adsorption d'oxygène, ce qui permet donc d'augmenter très notablement la capacité de stockage du gaz au sein de de la cartouche 1.

Par exemple, à une pression de 10 bar abs., une cartouche 1 de 660 ml de volume interne 12 peut contenir environ 400 g d'adsorbant 14-1 et, par conséquent, adsorber environ 12 L d'$O_2$, alors que l'espace interstitiel autour des particules d'adsorbant 14-1 définit un volume « vide » dans lequel des molécules d'$O_2$ vont également être comprimées et stockées, permettant ainsi d'augmenter la capacité totale à environ 15 L d'$O_2$ (à pression atmosphérique). A titre comparatif, en l'absence d'adsorbant 14-1, on ne pourrait y stocker qu'environ 6 L d'$O_2$, soit 2,5 fois moins.

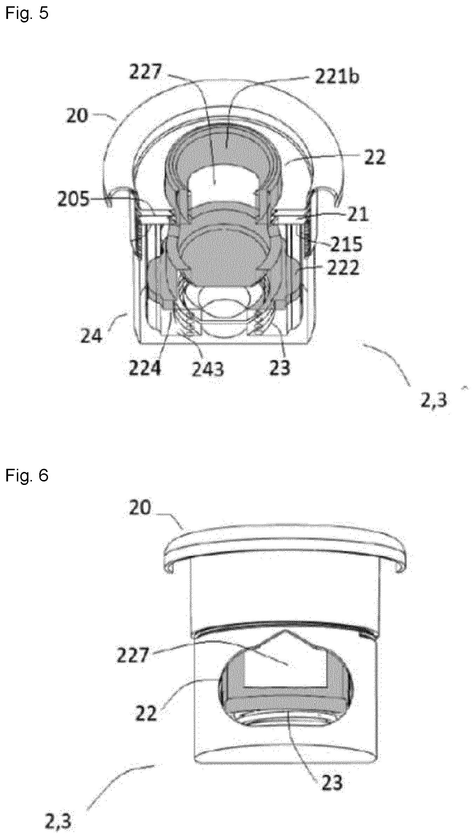

Fig. 5 et Fig. 6 représentent une configuration (vues partiellement tronquées) où ladite première valve 2 est en position « actionnée », c'est-à-dire lorsqu'une force extérieure vient s'appliquer sur la partie avant 221 de la pièce mobile 22, par exemple lors du raccordement d'un connecteur ou analogue, comme expliqué ci-après.

Dans ce cas, on voit que la pièce mobile 22 ne repousse plus le joint plat 21 contre le fond plat 205 du corps de valve 20 et que l'étanchéité fluidique est alors rompue, ce qui permet un passage de gaz au travers de ladite première valve 2.

Autrement dit, la pièce mobile 22 a subi une translation axiale vers l'intérieur de la cartouche 1, c'est-à-dire en direction de la pièce-couvercle 24, ce qui a pour conséquence de comprimer le ressort 23 vers le fond de la pièce-couvercle 24 tout en libérant le passage du gaz entre le joint plat 21 et le fond plat 205 du corps de valve 20.

La translation de la pièce mobile 22 en éloignement par rapport au corps de valve 20 engendre une perte de contact entre la face avant de la collerette 222 et le joint plat 21 et il se crée alors une communication fluidique entre l'extérieur et l'intérieur de la cartouche 1, via la première valve de contrôle 2, notamment au travers des ouvertures latérales 227 de la pièce mobile 22 et des ouvertures latérales 242 de la pièce-couvercle 24. Bien entendu, la seconde valve 3 fonctionne sur le même principe.

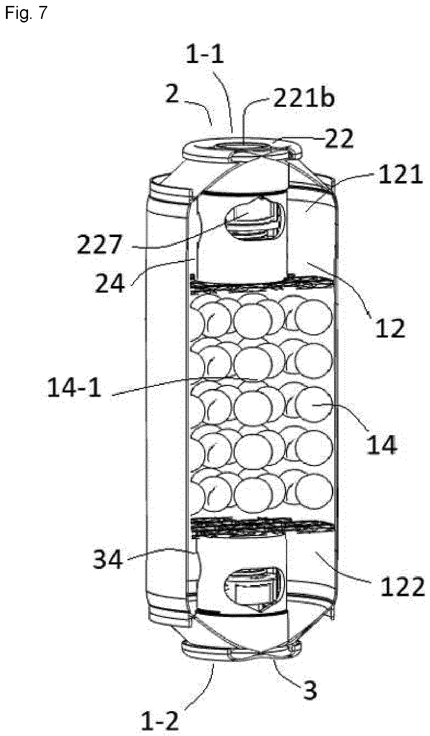

Fig. 7 montre la cartouche de stockage d'oxygène 1 avec ses deux valves de contrôle 2, 3 en position « actionnée », la structure et le fonctionnement de la deuxième valve de contrôle 3 étant identique à ceux de la première valve de contrôle 2.

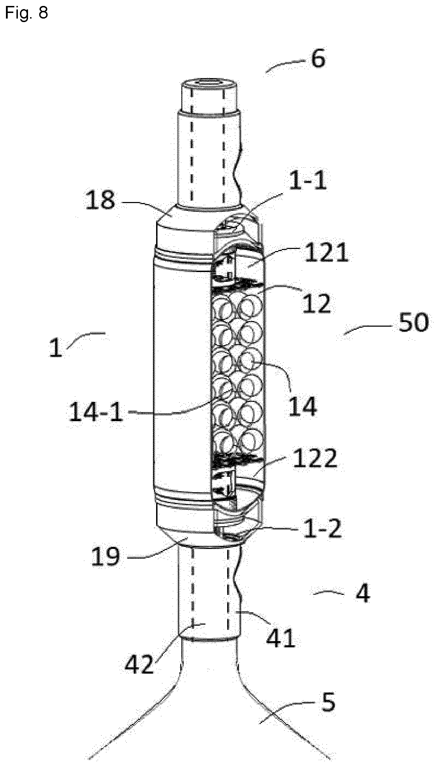

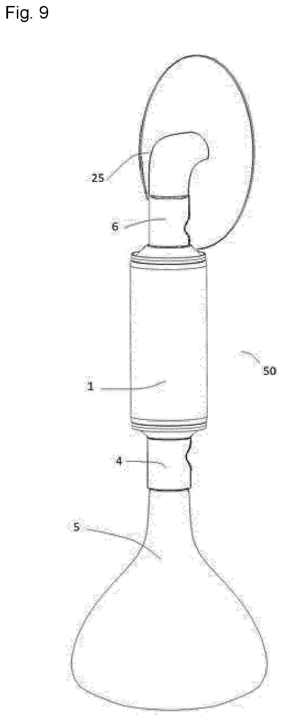

Les valves de contrôle 2, 3 sont dans une telle position « actionnée », lorsque la cartouche de stockage d'oxygène de Fig. 1 a été raccordée par un utilisateur à des dispositifs extérieurs, par exemple à, d'une part, un premier conduit de gaz 4 et, d'autre part, un second conduit de gaz 6, comme illustré sur Fig. 8, en formant ainsi un ensemble 50 de distribution d'oxygène de secours, comme illustré en Fig. 9.

Comme on le voit en Fig. 8, le premier conduit 4 comprend une enveloppe cylindrique 41 disposant d'un conduit interne creux 42 ou lumen, ayant une forme de cylindre creux.

Le premier conduit 4 est par ailleurs connecté fluidiquement un réservoir 5 déformable ou flexible, (représenté de façon partielle sur Fig. 8 et Fig. 9) vide de gaz, au moment du raccordement à la cartouche 1, mais pouvant d'être rempli, i.e. gonflé, d'un volume de gaz donné après raccordement du premier conduit 4 à la cartouche 1. Par exemple, le réservoir 5 peut être configuré pour collecter un volume de gaz supérieur ou égal au volume de gaz contenu dans le volume intérieur 12 de cartouche de gaz 1, i.e. $O_2$. Le réservoir 5 peut être formée d'une enveloppe souple en polymère. Le premier conduit 4 est raccordé fluidiquement, de manière étanche, par exemple au fond 19 la cartouche 1, par exemple par clipsage, vissage ou autre, portant la seconde valve 3.

Le conduit interne 42 du premier conduit 4 va alors agir sur la pièce mobile 22 de la valve de contrôle 3 pour la repousser vers l'intérieur de la cartouche 1 de sorte que la seconde valve 3 se retrouve en position « actionnée », avec rupture de l'étanchéité au niveau du joint plat de de la valve de contrôle 3, comme expliqué ci-dessous.

Il s'opère alors un transfert d'$O_2$ depuis le volume intérieur 12 du récipient de gaz 1 vers le premier conduit 4 puis vers le réservoir 5 qui augmente progressivement en volume, c'est-à-dire se remplit d'oxygène. Une fois le réservoir 5 rempli, il s'établit un équilibre de pression entre le réservoir 5 flexible et la cartouche de gaz 1, cette dernière se retrouvant alors à pression atmosphérique (i.e. 1 atm).

En procédant de la même manière, l'utilisateur peut raccorder un second conduit 6 à la valve 2 équipant le couvercle 18, ce qui, là encore, va repousser la pièce mobile 22 pour faire passer la première valve 2 dans sa position « actionnée », comme décrit ci-dessus, et établir alors une connexion fluidique entre le conduit interne du second conduit 6, le volume interne 12, le conduit interne 42 du premier conduit 4 et ensuite, par extension, le réservoir 5.

Le second conduit 6 peut par exemple alimenter une interface respiratoire 25, tel un masque respiratoire, visible en Fig. 9, qui peut être nasal ou facial (i.e. naso-buccal), c'est-à-dire que la cartouche 1 est alors prête à l'emploi par l'utilisateur, lequel peut inhaler de l'oxygène provenant de la cartouche 1 et/ou du réservoir 5.

De préférence, on choisit une interface respiratoire 25, tel un masque respiratoire étanche, c'est-à-dire comprenant des moyens d'étanchéité sur le pourtour de l'ouverture par laquelle l'utilisateur respire, c'est-à-dire l'ouverture arrière du masque recevant le nez (masque nasal) ou le nez et la bouche de l'utilisateur (masque facial), par exemple des moyens d'étanchéité de type coussin flexible ou analogue venant au contact des zones du visage de l'utilisateur entourant son nez et/ou sa bouche, afin d'y créer une étanchéité fluidique.

Ainsi, lors d'une phase inspiratoire de l'utilisateur, une partie de l'$O_2$ contenu dans le réservoir flexible 5, ressort de celui-ci puis transite successivement dans le conduit interne 42 du premier conduit 4, le volume intérieur 12 de la cartouche 1 au travers du lit d'adsorbant 14 et ensuite ressortir du volume intérieur 12 via le second conduit 6 et atteindre enfin le masque respiratoire 25 porté l'utilisateur.

A l'inverse, lors d'une phase expiratoire de l'utilisateur, une partie de l'$O_2$ inhalé (env. 5% vol.) a été substituée par du $CO_2$ et le gaz exhalé par l'utilisateur contient donc du $CO_2$, de la vapeur d'eau et une haute concentration d'$O_2$, c'est-à-dire plus de 90 % en volume. Le gaz expiré va suivre le chemin inverse à celui suivi par l'oxygène inhalé, à savoir emprunter le second conduit 6, traverser la première valve 2 pour atteindre alors le volume intérieur 12 de la cartouche 1, et transiter ensuite successivement au travers du lit d'adsorbant 14, puis de la seconde valve 3 et du premier conduit 4 pour atteindre enfin le réservoir déformable 5.

Une caractéristique de l'adsorbant 14-1 choisi, telles des particules de zéolite, est que sa capacité d'adsorption diffère selon les molécules considérées. Ainsi, la capacité d'adsorption de l'$O_2$ est relativement faible par rapport à la capacité d'adsorption du $CO_2$ ou de la vapeur d'eau, typiquement de 50 à 100 fois plus importante. Pour une concentration de 5% de $CO_2$, c'est-à-dire 5 kPa en pression absolue, la capacité d'adsorption d'une zéolite, par exemple de type Zeochem Z10-ZZ ou 5A, présente ainsi une capacité de stockage en volume similaire au volume total d'$O_2$ initialement présent dans la cartouche de gaz 1, mesurée à 10 bar. Cette capacité est encore plus importante pour la vapeur d'eau.

Dès lors, lorsque le gaz expiré par l'utilisateur traverse le premier adsorbant 14-1, les molécules de $CO_2$ et la vapeur d'eau vont être adsorbées de sorte que le gaz est débarrassé de la quasi-totalité du $CO_2$ (et de la vapeur d'eau) qu'il contient, c'est-à-dire qu'il s'agit d'oxygène quasi-pur. Cet oxygène gazeux exempt de $CO_2$, qui retourne dans le réservoir déformable 5 peut ensuite être inspiré à nouveau par l'utilisateur, lors d'une phase inspiratoire suivante.

A titre indicatif, la consommation métabolique en oxygène d'un individu est au maximum de 0.5 L/min, ce qui implique qu'après 10 minutes d'inhalation/expiration répétées, le réservoir 5 contient environ 5L d'$O_2$ en moins, dans les conditions susmentionnées, à savoir une pression de 10 bar et un volume du récipient de 660 ml. Ceci permet de subvenir aux besoins respiratoires d'un utilisateur pendant plusieurs dizaines de minutes.

Bien entendu, on peut utiliser plusieurs adsorbants différents, par exemple plusieurs lits successifs de différents adsorbants, à savoir zéolitiques ou autres.

Un tel ensemble 50 de distribution d'oxygène de secours comprenant une cartouche 1 de stockage d'oxygène portative selon l'invention raccordée, d'une part, à un réservoir 5 et, d'autre part, à une interface respiratoire 25, tel un masque, peut être utilisé pour fournir de l'oxygène à une personne, pendant un temps suffisant, dans une situation particulière engendrant une atmosphère néfaste, hypoxique et/ou chargée de composés délétères.

Le réservoir 5 et l'interface respiratoire 25 vient se raccorder mécaniquement à la cartouche 1 de stockage d'oxygène tout en assurant une continuité fluidique entre eux, via des moyens de raccordement adaptés, comme par exemple des connexions mâle/femelle par emboitement, par système vissé ou à baïonnette, ou autre.

Toutefois, comme expliqué ci-avant, un tel ensemble 50 de distribution d'oxygène de secours ne permet pas d'apprécier facilement le niveau de saturation de l'adsorbant 14-1, captant le $CO_2$ contenu dans le gaz expiré par l'utilisateur, lors de ses phases expiratoires répétées. En effet, au fur et à mesure du temps et des phases expiratoires qui se succèdent, la quantité de $CO_2$ retenue par le premier adsorbant, typiquement des particules de zéolites, augmente progressivement, c'est-à-dire que le $CO_2$ s'accumule petit à petit dans le premier lit d'adsorbant 14.

Potentiellement, cette quantité de $CO_2$ pourrait excéder la capacité d'adsorption dudit premier adsorbant 14-1 et de fait tout ou partie du $CO_2$ expiré pourrait circuler à travers la seconde valve 3, le premier conduit 4 pour atteindre enfin le réservoir déformable 5 et s'y accumuler, conduisant alors à un risque non négligeable d'intoxication et/ou d'hypoxie pour l'utilisateur.

Or, étant donné que le corps de cartouche 13 de la cartouche de stockage d'$O_2$ 1, qui est typiquement formé d'une enveloppe tubulaire 11, typiquement de forme cylindrique, est généralement en métal, par exemple en un alliage d'aluminium, et qu'il en va de même des premier 18 et second 19 couvercles qui prennent le corps cylindrique 13 en « sandwich » et y sont fixés de manière étanche, l'ensemble de la cartouche 1 est alors opaque, donc empêche toute visualisation de l'adsorbant 14 par l'utilisateur. De tout manière, le premier adsorbant 14-1, qui est de particules de zéolite ou analogue, ne subit aucune modification perceptible visuellement, c'est-à-dire de forme, texture et/ou couleur, lorsqu'il devient progressivement saturé en $CO_2$, et ne pourrait dès lors pas permettre à l'utilisateur de déterminer visuellement son niveau de saturation.

De là, selon l'invention, on propose de modifier la cartouche 1 décrite ci-dessus afin de renforcer la sécurité liée à la mise à disposition de l'ensemble 50 de distribution d'$O_2$ de secours, et donc permettre à un utilisateur de déterminer, facilement et en temps (quasi) réel, le niveau de saturation de la cartouche de stockage d'$O_2$ 1 et de pouvoir anticiper son remplacement avant que tout ou partie du $CO_2$ expiré et se retrouvant piégé sur le premier adsorbant 14-1, typiquement venant s'adsorber sur ledit premier adsorbant, ne se retrouve dans le réservoir 5, posant alors une problématique d'intoxication ou analogue.

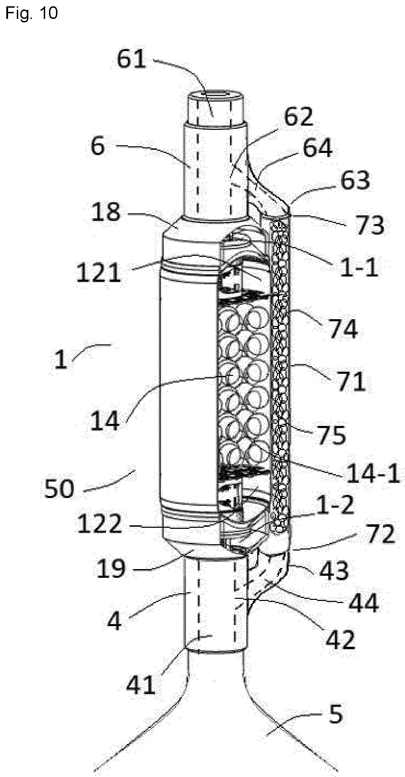

Pour ce faire, comme illustré en Fig. 10 à Fig. 12, l'invention propose une cartouche de stockage d'$O_2$ 1 améliorée dont l'architecture et le fonctionnement sont globalement identiques à ceux des Fig. 1 à Fig. 9, c'est-à-dire comme décrit ci-avant, mais mettant en œuvre une ligne de bipasse 71, dont l'architecture, le rôle et le fonctionnement sont décrits ci-après. Les éléments communs ou identiques ne sont pas répétés puisque déjà détaillés ci-dessus ; il suffit de s'y reporter.

Plus précisément, selon l'invention, comme illustré sur Fig. 10 à Fig. 12, on équipe la cartouche de stockage d'$O_2$ 1 d'une ligne de bipasse 71, aussi appelée ligne de dérivation, tel un tube allongé, venant se raccorder fluidiquement en des sites de raccordement 42, 62 situés en amont des premier et second orifices de gaz 1-1 ; 1-2, c'est-à-dire dont les extrémités viennent se raccorder sur le trajet du gaz, en dehors de la cartouche 1, de manière à ce qu'une partie du gaz, typiquement le gaz riche en $CO_2$ expiré par l'utilisateur, entrant dans le volume interne 12 de la cartouche 1, par l'un ou l'autre des premier et second orifices de gaz 1-1 ; 1-2, soit dérivée et circule au sein de cette ligne de bipasse 71.

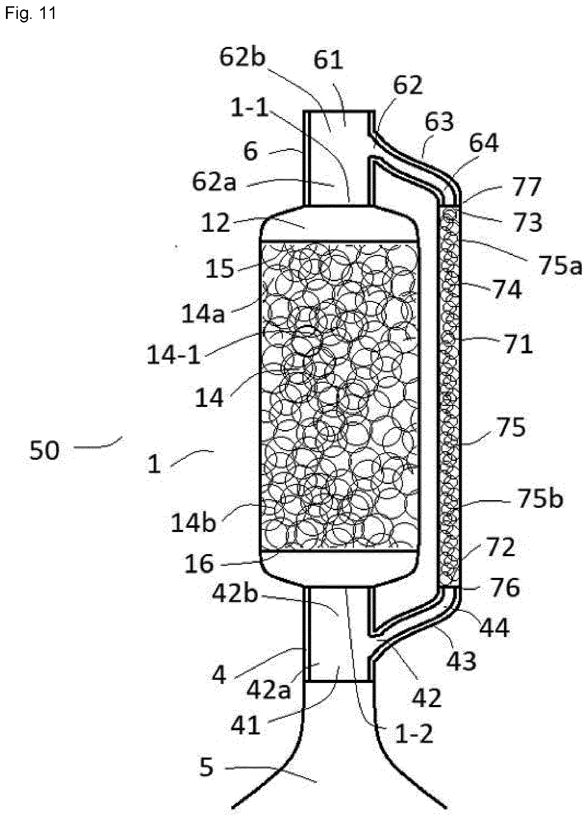

Autrement dit, une partie du gaz circulant entre l'interface respiratoire 25, cartouche 1 et le réservoir 5 (cf. Fig. 11), est dérivée et bipasse la cartouche 1 de stockage d'$O_2$ en circulant au sein de cette ligne de bipasse 71 et non plus au sein de la cartouche 1.

Comme visible en Fig. 10 à Fig. 12, la ligne de bipasse 71 vient préférentiellement se raccorder en des sites de raccordement 42, 62 localisés entre la cartouche 1 et, d'une part, le réservoir de gaz 5 et, d'autre part, l'interface respiratoire 25 de l'ensemble 50 de distribution d'oxygène de secours selon l'invention, c'est-à-dire des sites situés en amont des premier et second orifices de gaz 1-1 ; 1-2, donc de part et d'autre de la cartouche 1.

De plus, comme détaillé ci-après, la ligne de bipasse 71 contient au moins un second matériau adsorbant 75 et au moins un matériau indicateur de saturation, de préférence mélangé l'un à l'autre.

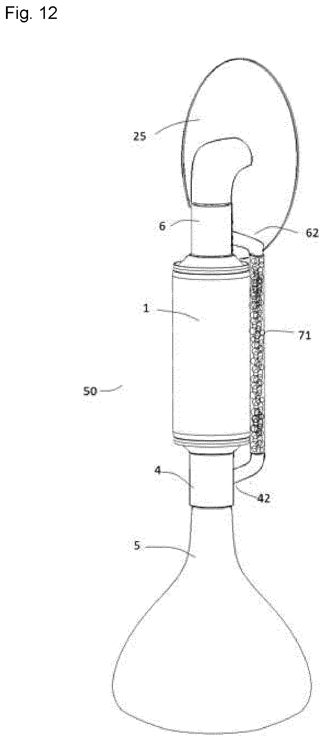

Plus précisément, Fig. 10 qui est semblable à la Fig. 7, à l'exception de la ligne de bipasse 71 supplémentaire, représente la cartouche 1 selon l'invention avec ses deux valves de contrôle 2, 3 en position « actionnée », après raccordement du premier conduit 4 à un réservoir 5 déformable et du second conduit 6 à une interface respiratoire 25, tel un masque nasal ou facial, comme illustré sur Fig. 12.

Comme illustré en Fig. 10, le premier conduit 4 comprend un premier conduit de dérivation 43 qui s'y raccorde fluidiquement en un premier site de branchement 42 de sorte que le lumen 41 du premier conduit 4 soit en communication fluidique avec le lumen 44 du premier conduit de dérivation 43.

De façon analogue, le second conduit 6 comprend un second conduit de dérivation 63 qui s'y raccorde fluidiquement en un second site de branchement 62 de sorte que le lumen 61 du second conduit 6 soit en communication fluidique avec le lumen 64 du second conduit de dérivation 63.

Les premier et second conduits de dérivation 43, 63 sont respectivement raccordés fluidiquement, par clipsage ou tout autre moyen, aux deux extrémités opposées 72, 73 de la ligne de bipasse 71, à savoir ici un long tube fin, aussi appelé tube de dérivation, formé d'un (ou plusieurs) matériau transparent, tel qu'un thermoplastique, par exemple du polycarbonate. Le volume interne 74 dudit tube de dérivation 71 est rempli d'un second adsorbant 75, comme décrit ci-après.

L'ensemble 50 de distribution d'oxygène de secours de la présente invention est représenté sous une forme simplifiée en Fig. 11. On comprend qu'il existe une relation fluidique entre le conduit interne ou lumen 61 du second conduit 6, le volume interne 12 de la cartouche de stockage d'$O_2$ 1, le conduit interne ou lumen 41 du premier conduit 4, et le réservoir flexible 5 de sorte que le gaz puisse circuler entre ces éléments du réservoir 5 jusqu'au masque 25 raccordé au second conduit 6, et inversement.

Le second conduit 6 présente un embranchement (en 62) prolongé par le second conduit de dérivation 63 qui communique fluidiquement avec une second extrémité 73 de la ligne de bipasse 71 et, de façon analogue, le premier conduit 4 présente un embranchement (en 42) prolongé par le premier conduit de dérivation 43 qui communique fluidiquement avec une première extrémité 72 de la ligne de bipasse 71.

La ligne de bipasse 71 comprend le second adsorbant 75 qui y est maintenu entre deux tamis 76, 77 qui le prennent en sandwich. Les tamis 77 de la ligne de bipasse 71 peuvent être analogues ou identiques aux premier et second tamis 15 et 16 agencés dans le corps de cartouche 11, de manière à permettre une circulation de gaz tout en empêchant au second adsorbant 75 de se déplacer dans la ligne de bipasse 71.

Il existe une relation fluidique entre les premier et second conduits 4, 6 et les premier et second conduits de dérivation 43, 63 de sorte qu'une portion du gaz expiré par l'utilisateur peut emprunter la ligne de bipasse 71 en passant au travers des deux tamis 76, 77 et du lit de second adsorbant 75 pris en sandwich entre eux.

Ainsi, lors d'une phase inspiratoire de l'utilisateur, une partie de l'$O_2$ contenu dans le réservoir flexible 5, ressort de celui-ci puis transite dans la portion amont 42a du premier conduit 4 pour ensuite se distribuer entre la portion avale 42b dudit conduit 4 et le premier conduit de dérivation 43 du premier conduit 4.

Une quantité majoritaire de gaz (i.e. > 80% du volume) circulant dans la portion avale 42b du premier conduit 4 peut alors pénétrer dans le volume intérieur 12 de la cartouche 1, passer au travers du premier lit d'adsorbant 14, avant de ressortir du volume intérieur 12 via la portion amont 62a du second conduit 62. Dans le même temps, une quantité minoritaire de gaz (i.e. < 20% du volume) circulant dans le premier conduit de dérivation 43 traverse l'adsorbant 75 contenu dans la ligne de bipasse 71, i.e. tube allongé transparent, emprunte le second conduit de dérivation 63 du second conduit 6 pour enfin déboucher, au niveau de l'embranchement 62, dans la portion avale 62b du second conduit 6.

Le flux de gaz circulant dans la portion aval 62b du second conduit 6 est donc la somme des flux gazeux provenant de la cartouche 1 et de la ligne de bipasse 71.

Ce flux de gaz chemine ensuite au travers du lumen du second conduit 6 pour ensuite arriver à l'interface respiratoire 25, tel un masque respiratoire porté l'utilisateur.

A l'inverse, lors d'une phase expiratoire de l'utilisateur, le gaz expiré va suivre le chemin contraire depuis l'interface respiratoire 25 jusqu'au réservoir 5 au travers de la cartouche 1, d'une part, et de la ligne de bipasse 71, i.e. tube allongé transparent, d'autre part. Ce gaz expiré contient du $CO_2$, de la vapeur d'eau et une concentration élevée d'$O_2$, typiquement plus de 90 % en volume, étant donné qu'une partie de l'$O_2$ inhalé (env. 5% vol.) a été substitué par du $CO_2$ dans les poumons de l'utilisateur. Le gaz atteint in fine le réservoir 5 où il réside jusqu'à la phase d'inhalation suivante.

La proportion de gaz transitant dans le premier adsorbant 14 contenu dans la cartouche 1 par rapport à celle transitant par le second adsorbant 75 contenu dans la ligne de bipasse 71, en phase inspiratoire et en phase expiratoire, dépend notamment des pertes de charges régnant des deux chemins empruntés par les flux de gaz, à savoir :

- le chemin de gaz principal comprenant notamment la cartouche 1 avec les premier et second tamis 15, 16 et le premier adsorbant 14, les première et seconde valve 2, 3 et une partie des conduits 4, 6.
- le chemin de gaz de dérivation comprenant les conduits de dérivation 63, 43, les tamis 77, 76, le second absorbant 75 et la ligne de bipasse 71.

Ainsi, en fonction des caractéristiques connues du chemin de gaz principal (i.e. sa résistance à l'écoulement d'un gaz), il est possible de dimensionner par modèle mathématique ou par expérimentation, le chemin de gaz de dérivation, c'est-à-dire la quantité de second adsorbant 75, le dimensionnement des conduits de dérivation 63, 43 et de la ligne de bipasse 71 afin de déterminer la quantité exacte de gaz transitant dans le chemin de gaz de dérivation. Par exemple, on peut s'assurer que 10% du gaz transite dans le chemin de gaz de dérivation et donc 90% dans le chemin de gaz principal, ou toute autre proportion.

Concernant les adsorbants utilisés, lorsque le premier adsorbant 14 contenu dans la cartouche de stockage d'oxygène 1 est de type zéolitique, par exemple la zéolite référencée Z10-ZZ commercialisée par la société Zeochem®, on peut choisir en tant que second adsorbant 75 contenu dans la ligne de bipasse 71, de la chaux sodée (i.e. composée majoritairement d'hydroxyde de calcium), préférentiellement un mélange contenant (au moins) de l'hydroxyde de calcium $(CaOH)_2$ et de l'éthyle violet en tant qu'indicateur coloré.

Un exemple d'un tel mélange adsorbant/indicateur coloré est commercialisé sous le nom de Litholyme™ par la société Allied Healthcare Product Inc. Ce mélange adsorbant/indicateur coloré se présente sous la forme de granules comprenant majoritairement de l'hydroxyde de calcium $(CaOH)_2$, i.e. > 95% en poids environ, du chlorure de lithium (LiCl), i.e. <3% en poids environ, et environ 1% en poids d'éthyle violet.

L'hydroxyde de calcium $(CaOH)_2$ du mélange adsorbant/indicateur coloré réagit qu'avec le $CO_2$ contenu dans le gaz expiré par l'utilisateur pour former du carbonate de calcium $CaCO_3$.

La réaction chimique est irréversible et induit une modification du pH auquel l'indicateur coloré de type « éthyle violet » ou analogue est sensible et tourne alors d'une couleur blanchâtre, tel un blanc cassé, à une couleur violette, tel un violet vif. Cette modification permet alors de visualiser l'état de chaque granule de chaux sodée.

Ainsi, lorsqu'un granule ne peut plus réagir avec le $CO_2$, sa couleur change progressivement d'un blanc cassé à un violet vif, ce qui permet de visualiser alors le taux de saturation. Autrement dit, l'indicateur coloré change de couleur en fonction du pH auquel il est soumis.

La capacité d'adsorption de $CO_2$ de ce type de granule est de l'ordre de 130 ml de $CO_2$ par g de produit, i.e. 130 ml/g. A titre comparatif, le premier adsorbant 14 a une capacité de 65 ml/g, soit deux fois moindre.

Le $CO_2$ n'étant présent que dans le gaz expiré par l'utilisateur, lors de ses phases expiratoires, seul le gaz circulant de l'interface 25 vers le réservoir 5 est de nature à saturer les premier et second adsorbants 14, 75. Lors des phases expiratoires, le $CO_2$ va être progressivement capté par le premier adsorbant 14, en particulier via sa portion amont 14a située en aval du premier tamis 15 jusqu'à ce qu'il soit saturé en $CO_2$, c'est-à-dire jusqu'à ce que le « front » de saturation qui se déplace, atteigne la portion aval 14b situé en regard du second tamis 16. Le premier adsorbant 14 arrive alors à saturation complète et ne peut plus adsorber de $CO_2$.

De la même manière, le $CO_2$ présent dans la partie de flux de gaz circulant dans la ligne de bipasse 71 va être capté par le second adsorbant 75, en particulier sa portion amont 75a située en aval du second tamis 77 jusqu'à ce que ladite portion 75a ne devienne saturée et que le « front » de saturation se déplace jusqu'à la portion aval 75b en amont du premier tamis 76. Le second adsorbant 15 est alors à saturation complète et ne peut plus adsorber de $CO_2$.

Selon l'invention, le « front de saturation » se propageant de façon progressive dans le second adsorbant 75 contenu dans la ligne de bipasse 71, il peut être visualisé au travers du matériau transparent formant la paroi de la ligne de bipasse 71, tel un tube transparent en polycarbonate ou analogue, par l'apparition progressive d'une coloration suivant le front de saturation, par exemple une coloration violette avec l'adsorbant susmentionné, i.e. les granules de Litholyme™.

Exemple

Pour un volume total de $CO_2$ $V_{CO2}$ contenu dans le gaz expiré par l'utilisateur se propageant dans la portion aval 62b du second conduit 6 et par ailleurs si « x » est le taux de diversion de ce volume dans le second conduit de dérivation 63, et donc (1-x) le complément circulant dans la portion amont 62a du second conduit 6, alors les quantités maximales respectives de $CO_2$ pouvant être captées par les premier et second adsorbants 14, 75 sont les suivantes :

$$x . V_{CO2} = C_{75} . M_{75} \quad \text{et} \quad (1-x) . V_{CO2} = C_{14} . M_{14}$$

Où :

- $C_{75}$ représente la capacité d'adsorption (ml/g) du second adsorbant 75,
- $M_{75}$ représente la masse (g) du second adsorbant 75,
- $C_{14}$ représente la capacité d'adsorption (ml/g) du premier adsorbant 14 et
- $M_{14}$ représente la masse (g) premier adsorbant 14.

Il en résulte alors que :

$$M_{75} = \frac{C_{14}.M_{14}}{C_{75}} \cdot \frac{x}{(1-x)}$$

Connaissant la capacité d'adsorption des deux adsorbants 14, 75, et en considérant une masse de premier adsorbant 14 de 500g, on obtient alors :

$$M_{75} = 250.\frac{x}{(1-x)}$$

Il apparait alors que la masse requise de second adsorbant 75 dépend du taux de diversion « x », mais que cette masse influe aussi elle-même sur le taux de diversion « x » car elle entraine une résistance à l'écoulement.

Dès lors, en utilisant des outils mathématiques ou en opérant des essais expérimentaux de routine, on peut pour déterminer la masse de second adsorbant ($M_{75}$) et le dimensionnement des différents éléments constituants le chemin de gaz de dérivation, c'est-à-dire notamment la ligne de bipasse 71, tel un tube de dérivation, les conduits de dérivation 43, 63, de sorte que l'équation précédente soit vérifiée.

En fait, la progression des fronts de saturation dans les adsorbants 14, 75 se fait de façon ratiométrique. Dit autrement, si l'on considère le niveau de saturation comme

étant de 0% lorsque le premier adsorbant 14 capte le $CO_2$ aux abords du premier tamis 15 et de façon similaire, lorsque le second adsorbant 75 capte le $CO_2$ aux abord de son second tamis 77, et de 100% lorsque le premier adsorbant 14 capte le $CO_2$ aux abords du second tamis 16 et donc le second adsorbant 75 captant le $CO_2$ aux abords de son premier tamis 76.

Dès lors, le second adsorbant 75 suit le même niveau de saturation que le premier adsorbant 14 au fur et à mesure des expirations successives de l'utilisateur, avec l'avantage de fournir à l'utilisateur une information visuelle du niveau de progression du front d'adsorption, marqué par l'apparition progressive d'une coloration violette de du second adsorbant 75.

Avantageusement, on peut prévoir un marquage gradué, c'est-à-dire des graduations ou analogues, par exemple sur le tube transparent formant la ligne de bipasse 71, permettant d'apprécier de façon plus simple et quantifiable le taux de saturation.

L'ensemble 50 de distribution d'oxygène de secours selon l'invention avec cartouche 1 de stockage d'O2 1 munie d'un indicateur visuel permet de mesurer en temps réel le taux de saturation du premier adsorbant 14 contenu dans la cartouche de stockage d'O2 1 et d'anticiper son remplacement avant que le $CO_2$ ne soit plus adsorbé et remplisse progressivement le réservoir 5.

## Revendications

1. Cartouche (1) de stockage d'oxygène portative comprenant :

   - un corps de cartouche (13) délimitant un volume interne (12) servant au stockage de l'oxygène et comprenant un premier orifice de gaz (1-1) et un second orifice de gaz (1-2) en communication fluidique avec le volume interne (12),
   - au moins une valve de contrôle de flux gazeux (2, 3) agencée dans chacun desdits premier et second orifices de gaz (1-1 ; 1-2) pour contrôler la circulation de gaz entrant dans le volume interne (12) et/ou sortant du volume interne (12) du corps de cartouche (13), et
   - au moins un lit d'adsorbant (14) contenant au moins un premier adsorbant (14-1) étant agencé dans le volume interne (12) du corps de cartouche (13), ledit premier adsorbant (14-1) présentant une capacité d'adsorption pour le $CO_2$ gazeux supérieure à une capacité d'adsorption pour l'oxygène gazeux,

   **caractérisée en ce que** :

   - elle comporte en outre au moins une ligne de bipasse (71) venant se raccorder fluidiquement en des sites de raccordement (42, 62) situés en amont de chacun desdits premier et second orifices de gaz (1-1 ; 1-2) de manière à ce qu'une partie du gaz entrant dans le volume interne (12) par l'un ou l'autre desdits premier et second orifices de gaz (1-1 ; 1-2) soit dérivée et circule au sein de ladite au moins une ligne de bipasse (71), et
   - au moins une partie de ladite au moins une ligne de bipasse (71) est formée d'un matériau transparent et contient au moins un second matériau adsorbant (75) et au moins un matériau indicateur de saturation.

2. Cartouche selon la revendication 1, **caractérisée en ce que** la cartouche (1) comprend une chambre centrale (124) contenant ledit au moins un lit d'adsorbant (14) contenant ledit au moins un premier adsorbant (14-1), une première chambre de recueil de gaz (121) et une seconde chambre de recueil de gaz (122), la chambre centrale (124) étant située entre les première et seconde chambres de recueil de gaz (121, 122).

3. Cartouche selon l'une des revendications précédentes, **caractérisée en ce que** ledit au moins un premier adsorbant (14-1) comprend des particules d'au moins une zéolite.

4. Cartouche selon la revendication 1, **caractérisée en ce que** ledit au moins un second matériau adsorbant (75) contenu dans la ligne de bipasse (71) comprend de la chaux sodée.

5. Cartouche selon la revendication 1, **caractérisée en ce que** ledit au moins un matériau indicateur de saturation contenu dans la ligne de bipasse (71) comprend un mélange contenant de l'hydroxyde de calcium et du violet d'éthyle.

6. Cartouche selon l'une des revendications 1, 4 ou 5, **caractérisée en ce que** lesdits au moins un second matériau adsorbant (75) et ledit au moins un matériau indicateur de saturation sont mélangés l'un à l'autre dans la ligne de bipasse (71).

7. Cartouche selon la revendication 6, **caractérisée en ce que** la ligne de bipasse (71) comprend un mélange particulaire contenant de l'hydroxyde de calcium $(CaOH)_2$, du violet d'éthyle et du chlorure de lithium (LiCl).

8. Cartouche selon la revendication 7, **caractérisée en ce que** la ligne de bipasse comprend au moins 95% en poids environ d'hydroxyde de calcium $(CaOH)_2$, moins de 3% en poids environ de chlorure de lithium (LiCl), et jusqu'à environ 1% en poids d'éthyle violet.

9. Cartouche selon l'une des revendications 6 à 8, **caractérisée en ce que** le matériau indicateur de saturation réagit en changeant progressivement de couleur au fur et à mesure du piégeage du $CO_2$ par les particules de chaux sodée engendrant une variation du pH.

10. Cartouche selon la revendication 9, **caractérisée en ce que** le matériau indicateur de saturation réagit en changeant progressivement de couleur en passant d'une couleur blanchâtre à une couleur violette.

11. Cartouche selon la revendication 1, **caractérisée en ce que** le matériau transparent est un polymère, de préférence un polycarbonate.

12. Cartouche selon la revendication 1, **caractérisée en ce que** la ligne de bipasse (71) comprend un marquage gradué.

13. Ensemble de distribution d'oxygène de secours (50) comprenant une cartouche (1) de stockage d'oxygène portative pour stocker de l'oxygène selon l'une des revendications précédentes, raccordée fluidiquement, via lesdits premier et second orifices de gaz (1-1 ; 1-2) de la cartouche (1), à un réservoir de gaz (5) et à une interface respiratoire (25).

14. Ensemble de distribution selon la revendication 13, **caractérisé en ce que** le réservoir de gaz est à enveloppe flexible.

15. Ensemble de distribution selon la revendication 13, **caractérisé en ce que** l'interface respiratoire est un masque respiratoire, en particulier un masque nasal ou facial.

**Patentansprüche**

1. Tragbare Sauerstoffspeicherpatrone (1), umfassend:

- einen Patronenkörper (13), der ein Innenvolumen (12) begrenzt, das der Speicherung von Sauerstoff dient und eine erste Gasöffnung (1-1) und eine zweite Gasöffnung (1-2) umfasst, die in Fluidverbindung mit dem Innenvolumen (12) stehen,
- mindestens ein Gasflusssteuerventil (2, 3), das in jeder der ersten und zweiten Gasöffnungen (1-1; 1-2) angeordnet ist, um die in das Innenvolumen (12) eintretende und/oder aus dem Innenvolumen (12) des Patronenkörpers (13) austretende Gaszirkulation zu steuern, und

- mindestens ein Adsorberbett (14), das mindestens ein erstes Adsorbens (14-1) enthält, das in dem Innenvolumen (12) des Patronenkörpers (13) angeordnet ist, wobei das erste Adsorbens (14-1) ein Adsorptionsvermögen für das gasförmige $CO_2$ aufweist, das höher als ein Adsorptionsvermögen für den gasförmigen Sauerstoff ist, **dadurch gekennzeichnet, dass**:

- sie ferner mindestens eine Bypassleitung (71) beinhaltet, die fluidisch an Anschlussstellen (42, 62) angeschlossen wird, die stromauf jeder der ersten und zweiten Gasöffnungen (1-1; 1-2) gelegen sind, so dass ein Teil des durch die eine oder die andere der ersten und zweiten Gasöffnungen (1-1; 1-2) in das Innenvolumen (12) eintretenden Gases abgezweigt wird und in der mindestens einen Bypassleitung (71) zirkuliert, und

- mindestens ein Teil der mindestens einen Bypassleitung (71) aus einem transparenten Material gebildet ist und mindestens ein zweites adsorbierendes Material (75) und mindestens ein Sättigungsanzeigematerial enthält.

2. Patrone nach Anspruch 1, **dadurch gekennzeichnet, dass** die Patrone (1) eine zentrale Kammer (124), die das mindestens eine Adsorberbett (14) enthält, welches das mindestens eine erste Adsorbens (14-1) enthält, eine erste Gassammelkammer (121) und eine zweite Gassammelkammer (122) umfasst, wobei die zentrale Gassammelkammer (124) zwischen den ersten und zweiten Gassammelkammern (121, 122) gelegen ist.

3. Patrone nach einem der vorhergehenden Ansprüche, **dadurch gekennzeichnet, dass** das mindestens eine erste Adsorbens (14-1) Partikel mindestens eines Zeoliths umfasst.

4. Patrone nach Anspruch 1, **dadurch gekennzeichnet, dass** das mindestens eine zweite adsorbierende Material (75), das in der Bypassleitung (71) enthalten ist, Atemkalk umfasst.

5. Patrone nach Anspruch 1, **dadurch gekennzeichnet, dass** das mindestens eine Sättigungsanzeigematerial, das in der Bypassleitung (71) enthalten ist, ein Gemisch umfasst, das Calciumhydroxid und Ethylviolett enthält.

6. Patrone nach einem der Ansprüche 1, 4 oder 5, **dadurch gekennzeichnet, dass** das mindestens eine zweite adsorbierende Material (75) und das mindestens eine Sättigungsanzeigematerial in der Bypassleitung (71) miteinander gemischt sind.

7. Patrone nach Anspruch 6, **dadurch gekennzeichnet, dass** die Bypassleitung (71) ein Partikelgemisch umfasst, das Calciumhydroxid $(CaOH)_2$, Ethylviolett und Lithiumchlorid (LiCl) enthält.

8. Patrone nach Anspruch 7, **dadurch gekennzeichnet, dass** die Bypassleitung mindestens etwa 95 Gew.-% Calciumhydroxid $(CaOH)_2$, weniger als etwa 3 Gew.-% Lithiumchlorid (LiCl) und bis zu etwa 1 Gew.-% Ethylviolett umfasst.

9. Patrone nach einem der Ansprüche 6 bis 8, **dadurch gekennzeichnet, dass** das Sättigungsanzeigematerial reagiert, indem es im Zuge des Entfernens des $CO_2$ durch die Atemkalkpartikel, was eine Änderung des pH-Werts bewirkt, zunehmend die Farbe wechselt.

10. Patrone nach Anspruch 9, **dadurch gekennzeichnet, dass** das Sättigungsanzeigematerial reagiert, indem es zunehmend die Farbe wechselt, wobei es von einer weißlichen Farbe zu einer violetten Farbe umschlägt.

11. Patrone nach Anspruch 1, **dadurch gekennzeichnet, dass** das transparente Material ein Polymer ist, bevorzugt ein Polycarbonat.

12. Patrone nach Anspruch 1, **dadurch gekennzeichnet, dass** die Bypassleitung (71) eine Skalenmarkierung umfasst.

13. Notsauerstoffabgabeanordnung (50), umfassend eine tragbare Sauerstoffspeicherpatrone (1) zum Speichern von Sauerstoff nach einem der vorhergehenden Ansprüche, die fluidisch, über die ersten und zweiten Gasöffnungen (1-1; 1-2) der Patrone (1), an einen Gasbehälter (5) und an eine Atemschnittstelle (25) angeschlossen ist.

14. Abgabeanordnung nach Anspruch 13, **dadurch gekennzeichnet, dass** der Gasbehälter mit flexiblem Gehäuse ausgeführt ist.

15. Abgabeanordnung nach Anspruch 13, **dadurch gekennzeichnet, dass** die Atemschnittstelle eine Atemmaske ist, insbesondere eine Nasen- oder Gesichtsmaske.

**Claims**

1. Portable oxygen storage cartridge (1) comprising:

   - a cartridge body (13) delimiting an internal volume (12) for storage of oxygen and comprising a first gas port (1-1) and a second gas port (1-2) in fluidic communication with the internal volume (12),
   - at least one gas flow control valve (2, 3) arranged in each of said first and second gas ports (1-1; 1-2) in order to control the flow of gas entering the internal volume (12) and/or exiting the internal volume (12) of the cartridge body (13), and
   - at least one adsorbent bed (14) containing at least one first adsorbent (14-1) and arranged in the internal volume (12) of the cartridge body (13), said first adsorbent (14-1) having an adsorption capacity for gaseous $CO_2$ greater than an adsorption capacity for gaseous oxygen,

   **characterized in that**:

   - it further comprises at least one bypass line (71) fluidically connected at connection sites (42, 62) located upstream of each of said first and second gas ports (1-1; 1-2) such that a portion of the gas entering the internal volume (12) through either of said first and second gas ports (1-1; 1-2) is diverted and flows within said at least one bypass line (71), and
   - at least a part of said at least one bypass line (71) is formed of a transparent material and contains at least one second adsorbent material (75) and at least one saturation indicator material.

2. Cartridge according to Claim 1, **characterized in that** the cartridge (1) comprises a central chamber (124) containing said at least one adsorbent bed (14) containing said at least one first adsorbent (14-1), a first gas collection chamber (121) and a second gas collection chamber (122), the central chamber (124) being located between the first and second gas collection chambers (121, 122).

3. Cartridge according to either of the preceding claims, **characterized in that** said at least one first adsorbent (14-1) comprises particles of at least one zeolite.

4. Cartridge according to Claim 1, **characterized in that** said at least one second adsorbent material (75) contained in the bypass line (71) comprises soda lime.

5. Cartridge according to Claim 1, **characterized in that** said at least one saturation indicator material contained in the bypass line (71) comprises a mixture containing calcium hydroxide and ethyl violet.

6. Cartridge according to any one of Claims 1, 4 and 5, **characterized in that** said at least one second adsorbent material (75) and said at least one saturation indicator material are mixed with each other in the bypass line (71).

7. Cartridge according to Claim 6, **characterized in that** the bypass line (71) comprises a particulate mixture containing calcium hydroxide $(CaOH)_2$, ethyl violet and lithium chloride (LiCl).

8. Cartridge according to Claim 7, **characterized in that** the bypass line comprises at least about 95% by weight calcium hydroxide $(CaOH)_2$, less than about 3% by weight lithium chloride (LiCl), and up to about 1% by weight ethyl violet.

9. Cartridge according to any one of Claims 6 to 8, **characterized in that** the saturation indicator material reacts by progressively changing colour as the $CO_2$ is trapped by the soda lime particles causing a change in pH.

10. Cartridge according to Claim 9, **characterized in that** the saturation indicator material reacts by progressively changing colour from a whitish colour to a violet colour.

11. Cartridge according to Claim 1, **characterized in that** the transparent material is a polymer, preferably a polycarbonate.

**12.** Cartridge according to Claim 1, **characterized in that** the bypass line (71) comprises a graduated marking.

**13.** Emergency oxygen delivery assembly (50) comprising a portable oxygen storage cartridge (1) for storing oxygen, according to any one of the preceding claims, fluidically connected, via said first and second gas ports (1-1; 1-2) of the cartridge (1), to a gas reservoir (5) and to a respiratory interface (25).

**14.** Delivery assembly according to Claim 13, **characterized in that** the gas reservoir is of the flexible envelope type.

**15.** Delivery assembly according to Claim 13, **characterized in that** the respiratory interface is a breathing mask, in particular a nose mask or face mask.

Fig. 1

Fig. 2

Fig. 3

Fig. 4

Fig. 5

Fig. 6

Fig. 7

Fig. 8

Fig. 9

Fig. 10

Fig. 11

Fig. 12

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2201224
- FR 2201227

- US 2021121649 A1